# EUROPEAN PATENT SPECIFICATION

(11) **EP 3 666 251 B1**
(45) Date of publication and mention of the grant of the patent: **06.09.2023**
(21) Application number: 18845159.5
(22) Date of filing: 03.07.2018
(51) Int. Cl.: A61K 8/81, A61Q 1/02, A61K 8/04, A61K 8/06, A61Q 17/04

(54) **POWDER-CONTAINING WATER-BASED COMPOSITION AND EXTERNAL SKIN PREPARATION**
PULVERHALTIGE ZUSAMMENSETZUNG AUF WASSERBASIS UND AUSSENHAUTVORBEREITUNG
COMPOSITION À BASE D'EAU CONTENANT DE LA POUDRE ET PRÉPARATION CUTANÉE EXTERNE

(30) Priority: 07.08.2017 JP 2017152391
(43) Date of publication of application: 17.06.2020
(73) Proprietor: Shiseido Company, Ltd., Tokyo 104-0061 (JP)
(72) Inventor: TERADA Tomoaki, Tokyo 104-0061 (JP); TAKAHASHI Kiyoshi, Tokyo 104-0061 (JP); MANAKA Yuta, Tokyo 104-0061 (JP); KIMURA Motoharu, Tokyo 104-0061 (JP)
(74) Representative: Ter Meer Steinmeister & Partner
(86) International application number: PCT/JP2018/025212
(87) International publication number: WO 2019/031112

(56) References cited:
- EP-A1- 1 216 693
- EP-A1- 2 786 735
- EP-A1- 3 539 532
- EP-B1- 1 216 693
- WO-A1-2017/073758
- WO-A1-2017/102359
- JP-A- 2013 116 870
- JP-A- 2014 037 404
- JP-A- 2015 003 885
- JP-A- 2015 120 682
- JP-A- 2015 124 149
- JP-A- 2016 169 324
- US-A- 5 419 848

## Description

### Cross Reference to Related Applications

The present invention is based upon and claims the benefit of the priority of Japanese Patent Application No. 2017-152391 (filed on August 7, 2017),

### Technical Field

The present disclosure relates to a powder-containing water-based composition including particles in a water-based solvent. Particularly, the present invention relates to a composition in which a powder is dispersed in a water-based solvent. The present disclosure also relates to an external skin preparation including this composition.

### Background Art

Products, such as cosmetics, inks, and ceramics, may employ compositions including particles in a solvent. For example, a cosmetic including a powder is known (see, for example, Patent Literatures 1 and 2).

Patent Literature 1 discloses a composition containing, in a cosmetically acceptable medium, at least: (a) at least one aqueous phase and (b) composite particles A in spherical form, having a mean particle size between 0.1 and 30 µm and including at least one type of particulate UV-screening agent and a core constituted of at least one type of inorganic material and/or of at least one type of organic material, and (c) free particles B of an inorganic UV-screening agent having a mean elementary particle size greater than 0.07 µm.

Patent Literature 2 discloses an oil-in-water-type makeup cosmetic including: (A) a crosspolymer including, as constituent units, 2-acrylamido-2-methylpropanesulfonic acid or a salt thereof, (meth)acrylic acid and/or an ester thereof, and N,N-dimethylacrylamide; (B) 0.2 to 3 mass%, with respect to an aqueous phase component, of one or more types of an inorganic salt and an organic salt; and (C) about 14 mass% of a powder. This oil-in-water-type makeup cosmetic includes about 20 mass% of an oil phase emulsified by a surfactant.

EP 2 786 735 discloses an oil-in-water-type emulsion cosmetic.

EP 1 216 693 discloses a gel-based eye shadow (A) containing at least one ammonium acryloyldimethyl taurate-vinyl pyrrolidone copolymer (I) and one or more dyes and/or colored pigments (II).

WO 2017/102359 discloses a composition for coating keratin materials, more particularly a composition of gel/gel type based on hydrophobic coated pigments and a liquid fatty acid and/or a glycol compound.

### Citation List

### Patent Literature

Patent Literature 1: Japanese Unexamined Patent Publication No. 2015-522046A
Patent Literature 2: Japanese Unexamined Patent Publication No. 2012-241003A

### Summary of Invention

### Technical Problem

Water-in-oil-based emulsion-type cosmetic compositions, such as disclosed in Patent Literature 1, are capable of achieving high powder dispersion stability, even when the content by percentage of powder is increased. Unfortunately, oil-based compositions tend to cause stickiness to be felt by the user. For example, oil-based compositions are not suitable for use in facial cleansing compositions, which are required to offer a refreshed feeling after use.

On the other hand, water-based compositions usually have difficulty in stably dispersing powder at a high content by percentage. The oil-in-water-type makeup cosmetic disclosed in Patent Literature 2 contains about 14 mass% of powder, but the content by percentage of the oil phase is high, and thus, like oil-based compositions, the cosmetic causes stickiness to be felt by the user. Removing the oil phase to eliminate stickiness will lead to loss of powder stability and dispersibility. Also, further increasing the content by percentage of powder in the oil-in-water-type makeup cosmetic disclosed in Patent Literature 2 will lead to loss of powder stability and dispersibility.

Thus, there is a demand for a water-based powder-containing composition having high powder dispersibility and stability even when the content by percentage of powder is high, and also having little stickiness.

### Solution to Problem

According to a first aspect of the present disclosure, a powder-containing water-based non-emulsified composition is provided, the composition according to Claim 1 comprising (A) an aqueous phase constituting a continuous phase, (B) 20 to 45% by mass of a powder, and (C) 0.7% by mass or greater of a thickener. The thickener is a polymer that includes 2-acrylamido-2-methylpropanesulfonic acid or a salt thereof as a constituent component. The powder is dispersed in the composition and the powder is at least one selected from the group consisting of synthetic mica, mica, sericite, talc, silica, barium sulfate, zinc myristate, glass powder, titanium dioxide, zinc oxide, boron nitride, silicone resin powder, polymethyl methacrylate powder, and nylon powder. A content of an oily component is 15% by mass or less relative to the mass of the composition.

According to a second aspect of the present disclosure, an external skin preparation comprising the composition according to the first aspect is provided.

### Advantageous Effects of Invention

The present invention can provide a water-based composition having high powder dispersibility and stability even when the content by percentage of powder is high.

In the composition of the present disclosure, the content by percentage of oily components can be kept low, even when the content by percentage of powder is high. Thus, stickiness can be suppressed at the time of application to the skin.

### Description of Embodiments

Preferred modes of the aforementioned aspects are described below.

According to a prefered mode of the above first aspect, the thickener includes at least one selected from a group of consisting of ammonium acryloyldimethyltaurate/beheneth-25 methacrylate crosspolymer, ammonium acryloyldimethyltaurate/vinylpyrrolidone copolymer, and ammonium acryloyldimethyltaurate/dimethylacrylamide crosspolymer.

According to a prefered mode of the above first aspect, the content by percentage of the thickener is 3% by mass or less relative to the mass of the composition.

According to a prefered mode of the above first aspect, two weeks after preparation, the composition has a viscosity of 20,000 mPa·s or greater.

According to a prefered mode of the above first aspect, thee composition further comprises 50% by mass or less of a water-soluble alcohol relative to the mass of the composition.

According to a prefered mode of the above first aspect, the content by percentage of the oily component is 5% by mass or less relative to the mass of the composition.

According to a prefered mode of the above first aspect, in case where the oily component is included, the content by percentage of a surfactant for emulsifying the oily component is 1% by mass or less relative to the mass of the composition.

According to a prefered mode of the above first aspect, the content by percentage of the aqueous phase is 40% by mass or greater relative to the mass of the composition.

According to a prefered mode of the above first aspect, the composition further comprises a volatile oily component. The powder is porous. The powder is impregnated with at least a portion of the oily component.

In the following description, POE is an abbreviation of polyoxyethylene, and POP is an abbreviation of polyoxypropylene. The number in parentheses after POE or POP indicates the average number of moles of POE groups or POP groups added in the compound in question.

The powder-containing water-based composition of the present disclosure includes (A) an aqueous phase constituting a continuous phase, (B) a powder, and (C) a thickener. "Water-based" means that the main phase is the aqueous phase, and does not mean that no oily component (oil phase) is included.

### {(A) Aqueous Phase}

The aqueous phase can act as a dispersion medium of the powder. The aqueous phase may include, for example, water, a water-soluble alcohol, or a mixture thereof. It is particularly preferred that the aqueous phase includes water.

The content by percentage of water relative to the mass of the composition may be 40% by mass or greater, 50% by mass or greater, 60% by mass or greater, or 70% by mass or greater. It is preferred to determine the content by percentage of water as appropriate depending on the purpose and use of the powder-containing water-based composition.

With respect to water, water used for such as cosmetics and quasi-pharmaceutical products can be used, including e.g., purified water, ion-exchanged water, and tap water. Depending on the purpose, the aqueous phase may further include a water-soluble alcohol.

Examples of water-soluble alcohols may include at least one type selected from lower alcohols, polyhydric alcohols, polyhydric alcohol polymers, dihydric alcohol alkyl ethers, dihydric alcohol alkyl ethers, dihydric alcohol ether esters, glycerin monoalkyl ethers, sugar alcohols, monosaccharides, oligosaccharides, polysaccharides, and derivatives of the above.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

In cases of employing the composition of the present disclosure in an external skin preparation, it is possible to suitably use, for example, ethanol, glycerin, propylene glycol, dipropylene glycol, or the like as the water-soluble alcohol.

The content by percentage of the water-soluble alcohol relative to the mass of the composition is, for example, preferably 50% by mass or less, more preferably 30% by mass or less, even more preferably 20% by mass or less. If the content by percentage of the water-soluble alcohol exceeds 50% by mass, the composition will have a strong alcohol smell. Also, irritation to the skin will become too strong in cases of employing the composition of the present disclosure in an external skin preparation.

The content by percentage of the aqueous phase relative to the mass of the composition may be 40% by mass or greater, 50% by mass or greater, 60% by mass or greater, or 70% by mass or greater. It is preferred to determine the content by percentage of the aqueous phase as appropriate depending on the purpose and use of the powder-containing water-based composition and on the content by percentage of other components.

### {(B) Powder}

The composition of the present disclosure includes at least one type of powder. The terms "powder" and "powdered component" as used herein are synonymous. The powder is not particularly limited so long as it is generally usable for cosmetic purposes, for example. Examples of the powder bodies may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc) and the like.

The powder's average particle size, specific surface area, specific gravity, particle shape, as well as whether the powder is porous or non-porous and whether it is subjected to surface treatment or not, can be chosen as appropriate depending on the purpose thereof. The powder may be a mixture of different types of powders.

The content by percentage of the powder relative to the mass of the composition is 20% by mass or greater. The content by percentage of the powder relative to the mass of the composition may be 25% by mass or greater, 30% by mass or greater, or 35% by mass or greater. If the content by percentage of the powder is less than 10% by mass, powder aggregation will increase, making it difficult to maintain a stable state. The content by percentage of the powder relative to the mass of the composition is 45% by mass or less, more preferably 40% by mass or less. If the content by percentage of the powder exceeds 45% by mass, viscosity will become too high, making it difficult to mix. For example, the content by percentage of the powder relative to the mass of the composition may be 35% by mass or less, 30% by mass or less, 25% by mass or less, or 20% by mass or less.

### {(C) Thickener}

The composition of the present disclosure includes a thickener. For the thickener, it is possible to use, for example, a taurate-based synthetic polymer and/or an acrylate-based synthetic polymer.

For the taurate-based polymeric thickener, it is possible to use, for example, polymers and/or copolymers (including crosslinked polymers) including 2-acrylamido-2-methylpropanesulfonic acid (acryloyldimethyl taurine acid) or a salt thereof (AMPS structure) as a constitutional unit. For such thickeners, it is possible to use, for example, at least one selected from an ammonium acryloyldimethyltaurate/beheneth-25 methacrylate crosspolymer (Aristoflex^{®} HMB from Clariant (Japan) K.K.), ammonium acryloyldimethyltaurate/vinylpyrrolidone copolymer (Aristoflex^{®} AVC from Clariant (Japan) K.K.), ammonium acryloyldimethyltaurate/carboxyethyl acrylate crosspolymer (Aristoflex^{®} TAC from Clariant (Japan) K.K.), polyacrylate crosspolymer-11 (Aristoflex^{®} Velvet from Clariant (Japan) K.K.), dimethylacrylamide/sodium acryloyldimethyltaurate crosspolymer, hydroxyethyl acrylate/sodium acryloyldimethyltaurate copolymer (SEPINOV EMT10 PINOV from Seppic), sodium acrylate/acryloyldimethyl taurine/dimethylacrylamide crosspolymer (SEPINOV P88 from Seppic), hydroxyethyl acrylate/sodium acryloyldimethyl taurate copolymer (SIMULGEL NS from Seppic), sodium acryloyldimethyltaurate/methacrylamidolauric acid copolymer (AMO-51 from Daitoh Chemical Co., Ltd.), and acrylamide/sodium acryloyldimethyltaurate/acrylic acid copolymer (Acudyne^{®} SCP from Dow Chemical Company).

Among the aforementioned thickeners, from the viewpoint of powder dispersibility and stability, it is preferred that the thickener includes at least one selected from ammonium acryloyldimethyltaurate/beheneth-25 methacrylate crosspolymer, ammonium acryloyldimethyltaurate/vinylpyrrolidone copolymer, and ammonium acryloyldimethyltaurate/dimethylacrylamide crosspolymer.

Examples of other thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride gellan gum, and Tremella fuciformis polysaccharide.

The content of the thickener relative to the mass of the composition is preferably 0.7% by mass or greater, more preferably 0.8% by mass or greater. If the thickener is less than 0.7% by mass, powder stability will deteriorate. The content of the thickener relative to the mass of the composition is preferably 3% by mass or less, more preferably 2% by mass or less, even more preferably 1.5% by mass or less. If the thickener exceeds 3% by mass, stickiness will increase at the time of application to the skin.

### {Oily Component}

The composition of the present disclosure may include an oily component. The composition of the present disclosure may be an oil-in-water type composition. There is no particular limitation to the oily component to be used in the present invention, so long as it is generally usable in external skin preparations and cosmetic products. Concrete examples may include liquid oils, solid fats, waxes, hydrocarbons, higher fatty acids, higher alcohols, synthetic ester oils, silicone oils, and perfumes. Note that, herein, "oily components" encompass oil components and components soluble in oil components.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, isododecane, isohexadecane, and the like.

Examples of the higher fatty asid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid (EPA), docosahexaenoic acid (DHA) and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

The content by percentage of the oily component relative to the mass of the composition may be, for example, 15% by mass or less, 10% by mass or less, or 5% by mass or less. It is possible that the composition contains substantially no oily component (0% by mass). The smaller the content of oily components, particularly nonvolatile oily components, the more preferable, in order to suppress stickiness at the time of application to the skin.

The oily component in the composition of the present disclosure is not emulsified. The composition of the present disclosure can ensure powder stability and dispersibility, even without an emulsified oily component. Thus, stickiness can be suppressed from arising.

The oily component can be added by impregnating a porous powder with the oily component. For example, by making a porous powder absorb a volatile oily component, such as a perfume, the oily component can be volatilized from the powder after application to the skin. Also, stickiness can be suppressed by impregnating the powder with the oily component. In the present disclosure, "volatile" means that the component can easily volatilize at room temperature, outside air temperature, and/or body temperature when applied to the skin.

The composition of the present disclosure may include, as appropriate and as necessary, other components-such as ester, anionic surfactants, cationic surfactants, amphoteric surfactants, nonionic surfactants, moisturizers, water-soluble polymers, film-forming agents, UV absorbers, metal ion sequestering agents, amino acids, organic amines, polymer emulsions, silicone elastomers, pH adjusters, skin nutrients, vitamins, antioxidants, antioxidant aids, and perfumes-in amounts that do not inhibit the effects of the present disclosure.

Examples of other components that may be blended are described below. At least one of the following components may be added to the composition of the present disclosure.

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat) ,glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer; and the like.

Examples of the ultraviolet light absorbers may include benzoic acid family ultraviolet light absorber (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc); anthranilic acid family ultraviolet light absorber (such as homomenthyl N-acetylanthranilate etc); salicylic acid family ultraviolet light absorber (such as amyl salicylate, menthyl salicylate, homomenthyl salicylate, octyl salicylate, phenyl salicylate, benzyl salicylate, p-isopropanolphenyl salicylate, etc); cinnamic acid family ultraviolet light absorber (such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc); benzophenone family ultraviolet light absorber (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc); 3-(4'-methylbenzylidene)-d,l-camphor and 3-benzylidene-d,l-camphor; 2-phenyl-5-methylbenzoxazol; 2,2'-hydroxy-5-methylphenylbenzotriazol, 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; 5-(3,3-dimethyl-2-norbornylidene)-3-pentane-2-one; dimorpholinopyridazinone; 2-ethylhexyl 2-cyano-3,3-diphenylacrylate; 2,4-bis-{[4-(2-ethylhexyloxy)-2-hydroxy]-phenyl}-6-(4-methoxyphenyl)-(1,3,5)-tria zine, and the like.

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure further may inculde, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

The content by percentage of the surfactant relative to the mass of the composition is, for example, preferably 5% by mass or less, more preferably 3% by mass or less, even more preferably 1% by mass or less, further more preferably 0.5% by mass or less. It is possible that the composition contains substantially no surfactant. It is preferred that the composition contains substantially no surfactant for emulsifying the oily component. By reducing the content by percentage of the surfactant, it is possible to suppress stickiness from arising when applying the composition to the skin.

The composition of the present disclosure can ensure powder stability and dispersibility, even without including an inorganic salt, such as sodium chloride, or an organic salt (other than the thickener), such as sodium citrate, for improving powder stability and dispersibility. The content by percentage of the aforementioned salt (other than the thickener) relative to the mass of the composition may be 0.5% by mass or less, 0.2% by mass or less, 0.1% by mass or less, or 0% by mass.

The viscosity of the composition of the present disclosure two weeks after preparation is preferably 8,000 mPa s or greater, more preferably 12,000 mPa s or greater, even more preferably 15,000 mPa·s or greater. A viscosity less than 8,000 mPa s will deteriorate usability. The viscosity of the composition is preferably 300,000 mPa s or less, more preferably 200,000 mPa s or less. A viscosity exceeding 300,000 mPa s will make it difficult to apply the composition of the present disclosure uniformly to the skin. The viscosity of the composition may be 150,000 mPa s or less, 100,000 mPa s or less, 80,000 mPa s or less, or 50,000 mPa s or less. The viscosity is preferably measured according to JIS Z8803.

An external skin preparation according to the present disclosure will be described. The external skin preparation of the present disclosure includes the powder-containing water-based composition of the present disclosure. The external skin preparation may include desired additives, such as those described above. Examples of external skin preparations may include face wash, skincare products, sun-block products, cosmetics, and fragrances.

With the powder-containing water-based composition and the external skin preparation according to the present disclosure, it is possible to increase the content by percentage of powder while maintaining high powder stability and dispersibility, even in a water-based composition. The powder stability and dispersibility are sustainable. In the composition of the present disclosure, since the content by percentage of the oil phase and surfactants can be reduced, stickiness can be suppressed at the time of application to the skin. By increasing the content by percentage of powder, the powder can remain on the skin even if the composition applied to the skin is wiped off. Various types of powders are applicable to the composition and the external skin preparation of the present disclosure, and thus, the type of powder can be selected depending on the purpose.

A method for manufacturing a powder-containing water-based composition and an external skin preparation according to the present disclosure will be described. The powder-containing water-based composition and the external skin preparation of the present disclosure can be manufactured by mixing an aqueous phase, a powder, and a thickener. For example, the powder-containing water-based composition and the external skin preparation of the present disclosure can be manufactured by: first dissolving a thickener in an aqueous phase; and then adding and mixing a powder to the aqueous phase.

In cases of impregnating a porous powder with an oily component, it is preferred to first impregnate the powder with the oily component and then adding the oily component-containing powder to a solvent (aqueous phase).

### Examples

### {Test Example 1}

A plurality of thickeners were studied as to whether they were capable of improving the stability of powder included at a high content by percentage in a water-based composition, without adding any oily component or surfactant. The formation of each prepared sample is shown in Table 1. The unit employed for indicating the content by percentage in the Table, including the following Test Examples, is percent by mass (mass%). Ion-exchanged water was used for the a plurality of types of powders (referred to hereinafter as "powder group") was used. Table 3 shows the contents by percentage of the powdered components employed in Powder Group A. Table 4 shows the contents by percentage of the powdered components employed in Powder Group B.

The results of visual verification of powder stability immediately after sample preparation and on the following day are shown in Table 5. "Syneresis" in Table 5 refers to a state in which water is floating on the upper surface or above the powder. "Aggregation" refers to a state in which the powder has gathered together and dispersibility has deteriorated.

In Test Examples 1-1 to 1-7, no deterioration in stability was verified. More specifically, in cases where the thickener is a polymer and/or a copolymer including 2-acrylamido-2-methylpropanesulfonic acid (acryloyldimethyl taurine) or a salt thereof (an AMPS structure) as a constituent unit and in cases where the thickener is an acrylate/steareth-20 methacrylate copolymer, no deterioration in powder stability was verified, even when the content by percentage of powder was as high as 40 mass%. On the other hand, the thickeners used in Test Examples 1-8 to 1-17 were incapable of stably dispersing a high content by percentage of powder. It is thus considered that thickeners including 2-acrylamido-2-methylpropanesulfonic acid or a salt thereof as a constituent component are useful in achieving stability of powder at a high content by percentage in a water-based composition.

As shown in Tables 3 and 4, Powder Group A and Powder Group B each include a plurality of types of powders. It is thus considered that powder dispersion stability is independent of the type of powder. Test examples for each type of powder will be described later in Test Examples 4.

**[Table 1]**

| | Test Example 1 |
|---|---|
| (A) Aqueous phase | 59.5 |
| (B) Powder | 40 |
| (C) Thickener | 0.5 |
| (A) Aqueous phase | 59.5 |
| (B) Powder | 40 |
| (C) Thickener | 0.5 |
| Total | 100 |

**[Table 2]**

| Test Exam ple | (C)Thickener | | (B)Powde r |
|---|---|---|---|
| 1-1 | Ammonium acryloyldimethyltaurate/beheneth-25 m ethacrylate crosspolymer ^{*1} | Thickener A | Powder Group A |
| 1-2 | Ammonium acryloyldimethyltaurate/beheneth-25 m ethacrylate crosspolymer ^{*1} | Thickener A | Powder Group B |
| 1-3 | Ammonium acryloyldimethyltaurate/vinylpyrrolidone copolymer *2 | Thickener B | Powder Group A |
| 1-4 | Polyacrylate crosspolymer-11 ^{*3} | Thickener C | Powder Group B |
| 1-5 | Ammonium acryloyldimethyltaurate/carboxyethyl acrylate crosspolymer ^{*4} | Thickener D | Powder Group B |
| 1-6 | Ammonium acryloyldimethyltaurate/dimethylacrylamide crosspolymer ^{*5} | Thickener E | Powder Group B |
| 1-7 | Acrylate/steareth-20 methacrylate copolymer ^{*6} | Thickener F | Powder Group B |
| 1-8 | Hydroxypropylcellulose ^{*7} | Thickener G | Powder Group A |
| 1-9 | Hydroxypropylcellulose ^{*8} | Thickener H | Powder Group A |
| 1-10 | Hydroxyethylcellulose ^{*9} | Thickener I | Powder Group A |
| 1-11 | Carboxyvinyl polymer ^{*10} (non-neutralized) | Thickener J | Powder Group B |
| 1-12 | Carboxyvinyl polymer ^{*10} (neutralized) | Thickener K | Powder Group A |
| 1-13 | Acrylates/C10-30 alkyl acrylate crosspolymer ^{*11} (neutralized) | Thickener L | Powder Group A |
| 1-14 | Sodium acrylate/vinylacetamide copolymer ^{*12} | Thickener M | Powder Group B |
| 1-15 | (PEG-240/decyltetradeceth-20/HDI) copolymer ^{*13} | Thickener N | Powder Group B |
| 1-16 | PEG-90M ^{*14} | Thickener O | Powder Group B |
| 1-17 | Tamarind gum ^{*15} | Thickener P | Powder Group B |
| 1-18 | Nothing added | - | Powder Group A |
| *1: Aristoflex (registered trademark) HMB from Clariant Japan K.K. | | | |
| *2: Aristoflex (registered trademark) AVC from Clariant Japan K.K. | | | |
| *3: Aristoflex (registered trademark) Velvet from Clariant Japan K.K. | | | |
| *4: Aristoflex (registered trademark) TAC from Clariant Japan K.K. | | | |
| *5: SU-GEL from Toho Chemical Industry Co., Ltd. | | | |
| *6: Aculyn (registered trademark) 22 from Dow Chemical Company | | | |
| *7: HPC-SSL from Nippon Soda Co., Ltd. | | | |
| *8: HPC-H from Nippon Soda Co., Ltd. | | | |
| *9: Natrosol 250HR from Hercules Incorporated | | | |
| *10: Carbopol (registered trademark) 981 from Lubrizol Advanced Materials, Inc. | | | |
| *11: Pemulen (registered trademark) TR2 from Lubrizol Advanced Materials, Inc. | | | |
| *12: adHERO from Showa Denko K.K. | | | |
| *13: Adeka Nol (registered trademark) GT-700 from Adeka Corporation | | | |
| *14: PEO-15P from Sumitomo Seika Chemicals Co., Ltd. | | | |
| *15: Glyloid 6C from Dainippon Pharmaceutical Co., Ltd. | | | |

**[Table 3]**

| Powder Group A | Content by percentage in powder group |
|---|---|
| Hydrophobized talc ^{∗16} | 24.8 |
| Talc ^{∗17} | 13 |
| Synthetic mica A ^{∗18} | 15 |
| Synthetic mica B ^{∗19} | 5 |
| Barium sulfate ^{∗20} | 5 |
| Titanium dioxide A ^{∗21} | 13 |
| Titanium dioxide B ^{∗22} | 10 |
| Yellow iron oxide ^{∗23} | 3 |
| Black iron oxide ^{∗24} | 0.2 |
| Crosslinked polymethyl methacrylate ^{∗25} | 4 |
| Spherical silica ^{∗26} | 4 |
| Zinc oxide ^{∗27} | 3 |
| *16: SA-JA-68R from Miyoshi Kasei Industry Co., Ltd. | |
| 17: Talc-15 from Asada Milling Co., Ltd. | |
| *18: PDM-9WA from Topy Industries Ltd. | |
| *19: PDM-5L from Topy Industries Ltd. | |
| *20: H-LFM from Sakai Chemical Industry Co., Ltd. | |
| *21: Tipaque (registered trademark) CR-50P from Ishihara Sangyo Kaisha, Ltd. | |
| *22: MT-100TV from Tayca Corporation | |
| *23: OTS-2 STN-1 from Daitoh Chemical Co., Ltd. | |
| *24: OTS-2 BL-100P from Daitoh Chemical Co., Ltd. | |
| *25: Techpolymer MPB-8HP from Sekisui Plastics Co., Ltd. | |
| *26: Sunsphere L51S from AGC Si-Tech Co., Ltd. | |
| *27: Zinc Oxide AZO-BS from Seido Chemical Industry Co., Ltd. | |

**[Table 4]**

| Powder Group B | Content by percentage in powder group |
|---|---|
| Hydrophobized talc ^{∗28} | 43.6 |
| Talc ^{∗17} | 15.3 |
| Sericite ^{∗29} | 10 |
| Powdered glass ^{∗30} | 3 |
| Zinc myristate ^{∗31} | 3 |
| Synthetic mica ^{∗19} | 5 |
| Hydrophobized titanium oxide ^{*21} | 3 |
| Titanium oxide ^{*22} | 7.5 |
| Red iron oxide ^{∗32} | 0.9 |
| Yellow iron oxide ^{*23} | 2.5 |
| Black iron oxide ^{∗24} | 0.2 |
| Powdered nylon ^{∗33} | 1 |
| Zinc-coated silicone ^{∗34} | 5 |
| *28: BAE-Talc JA-68R from Miyoshi Kasei Industry Co., Ltd. | |
| *29: Sericite FSE from Sanshin Mining Ind. Co., Ltd. | |
| *30: Silky Flake (registered trademark) FTD 10 from Nippon Sheet Glass Co., Ltd. | |
| *31: Zinc Myristate from Nippon Oil and Fats Co., Ltd. | |
| *32: OTS-2 Colcothar No. 216P from Daitoh Chemical Co., Ltd. | |
| *33: SP-500 from Toray Industries, Inc. | |
| *35: KSP100 from Shin-Etsu Silicone | |

**[Table 5]**

| Test Example | Immediately after sample prep aration | Following day |
|---|---|---|
| 1-1 | No problem | No problem |
| 1-2 | No problem | No problem |
| 1-3 | No problem | No problem |
| 1-4 | No problem | No problem |
| 1-5 | No problem | No problem |
| 1-6 | No problem | No problem |
| 1-7 | No problem | No problem |
| 1-8 | Syneresis | Syneresis |
| 1-9 | No problem | Slight syneresis |
| 1-10 | No problem | Slight syneresis |
| 1-11 | Syneresis (aggregation) | Syneresis |
| 1-12 | No problem | Syneresis (aggregation) |
| 1-13 | No problem | Syneresis (aggregation) |
| 1-14 | Slight syneresis | Slight syneresis |
| 1-15 | Syneresis | Syneresis |
| 1-16 | Syneresis | Syneresis |
| 1-17 | Syneresis | Syneresis |
| 1-18 | Syneresis | Syneresis |

### {Test Example 2}

As in Test Example 1, compositions with different types of thickeners were prepared, and the powder stability and dispersibility of each composition were observed and also the viscosity was measured. Synthetic mica was used for the powder. The content by percentage of the thickener was different from that in Test Example 1. Further, in Test Example 2, the aqueous phase included alcohols. The evaluation criteria for stability and dispersibility are described below. The viscosity was measured immediately after preparation of the composition and two weeks after preparation at 30°C with a rotary viscometer (BH-type viscometer from Brookfield) using Rotor No. 6 at a rotation speed of 10 rpm. The formations and results are shown in Tables 6 to 8. In Tables 6 to 8, for the sake of brevity of indication, the thickeners are indicated using the symbols shown in Table 2. EDTA 3Na·2H₂O refers to a trisodium salt of ethylenediaminetetraacetic acid.

### {Stability (after two weeks)}

∘: No powder settling nor syneresis.
Δ: No powder settling but occurrence of syneresis.
×: Both powder settling and syneresis.

### {Dispersibility}

∘: No unevenness.
Δ: Slight unevenness observed.
×: Uneven dispersion, creating an uneven-colored pattern.

The thickeners (except for Thickener F) that caused no problem in stability in Test Example 1 also caused no problem in Test Example 2, neither in terms of powder stability nor dispersibility. The thickeners that caused problems in Test Example 1 were particularly unable to achieve good powder dispersibility. Thickener F caused no problem in Test Example 1, but caused a problem in terms of dispersibility in Test Example 2. Thus, it was found that thickeners having an AMPS structure are effective in terms of dispersibility of powder at a high content by percentage.

In Test Examples 2-18 to 2-21, the same thickeners as those used in Test Examples 2-12 and 2-13 were used, but the content by percentage of the thickeners were increased compared to Test Examples 2-12 and 2-13. Although the viscosity increased, no improvement in dispersibility was observed. Thus, it is considered that powder dispersibility is dependent on the type of thickener, rather than viscosity.

In contrast to Test Example 1, the aqueous phase in Test Example 2 included alcohols, but this did not affect powder stability and dispersibility.

The compositions of Test Example 2 included no oily component, but were able to sustain powder stability for two weeks even when the action of emulsification was not employed. Since the compositions of Test Example 2 include no oily component nor surfactant, it is considered that stickiness is suppressed from arising at the time of application to the skin.

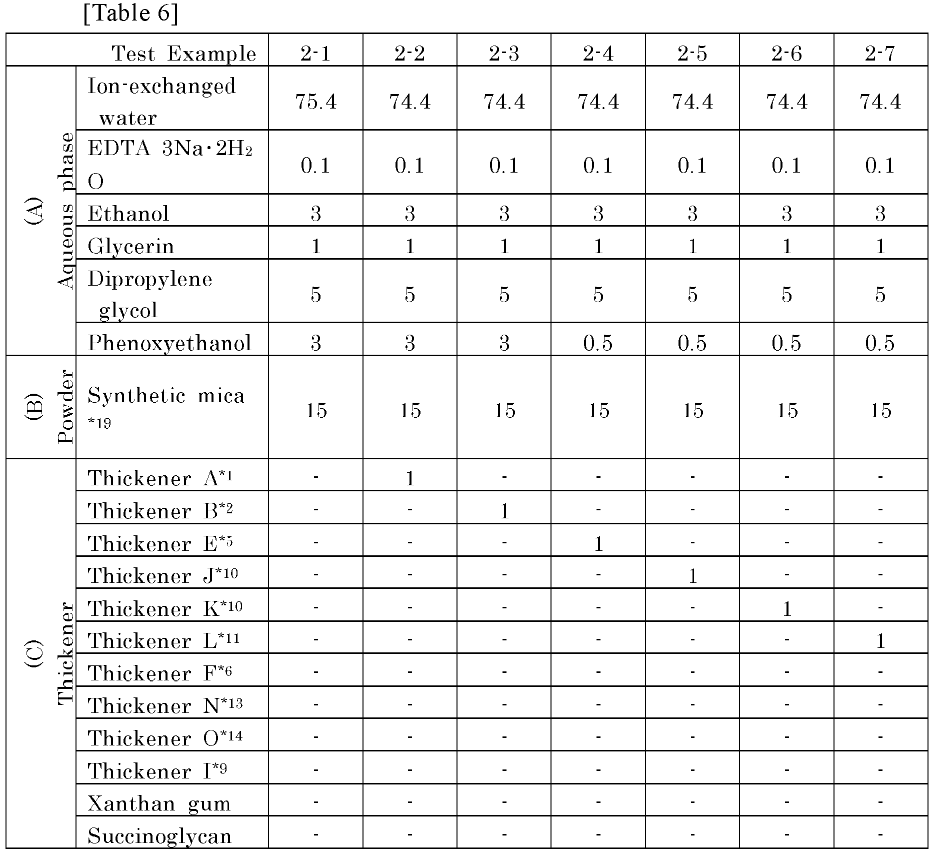

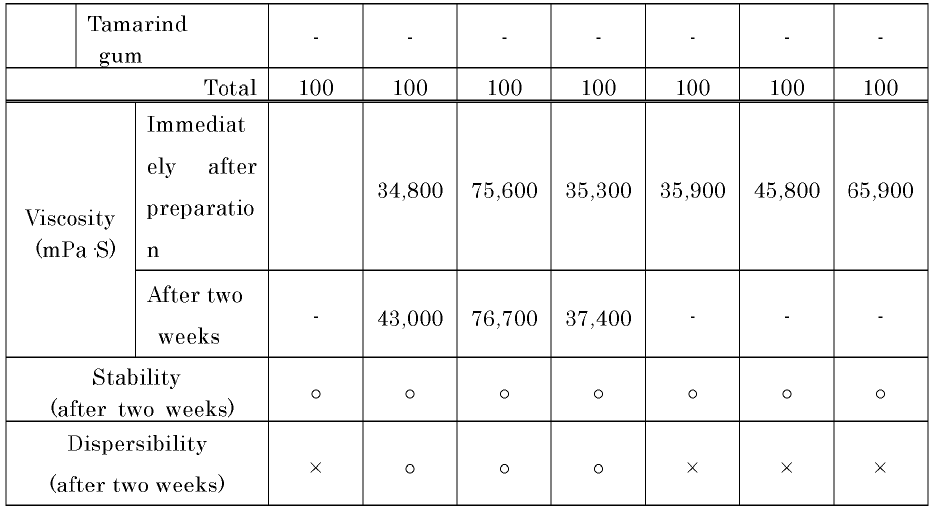

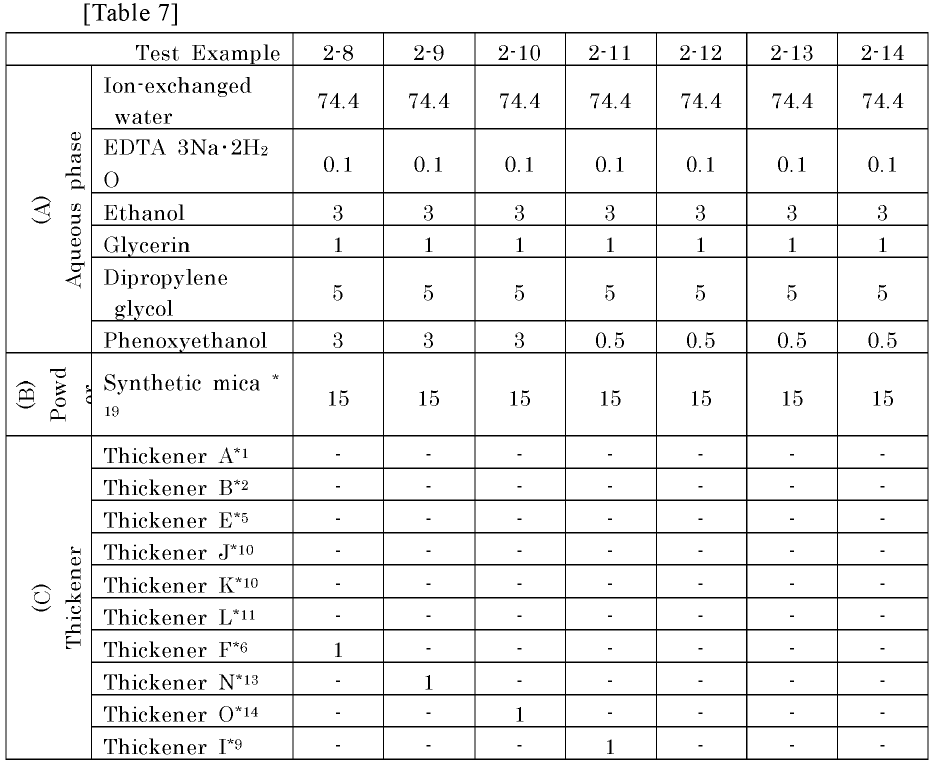

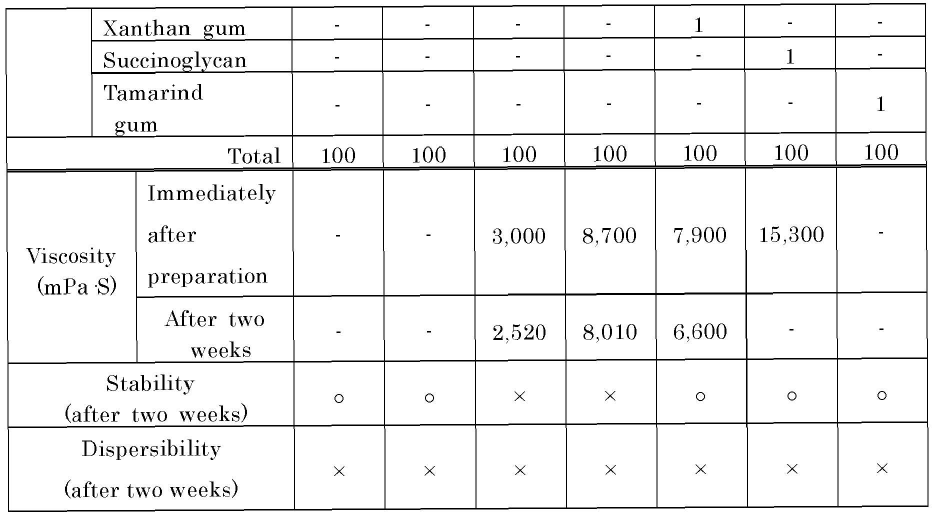

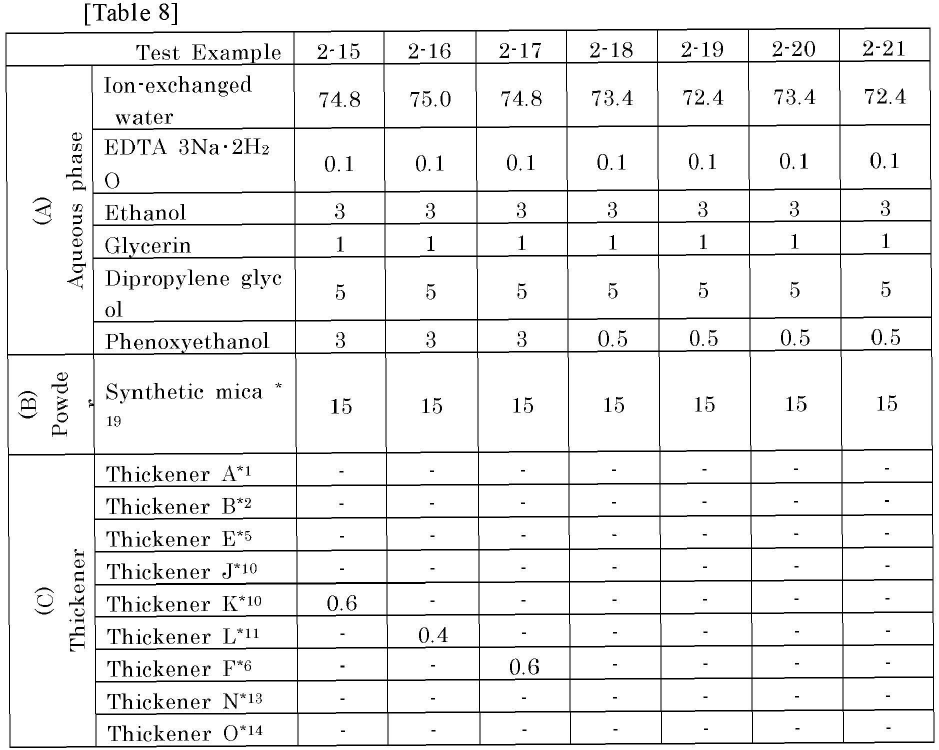

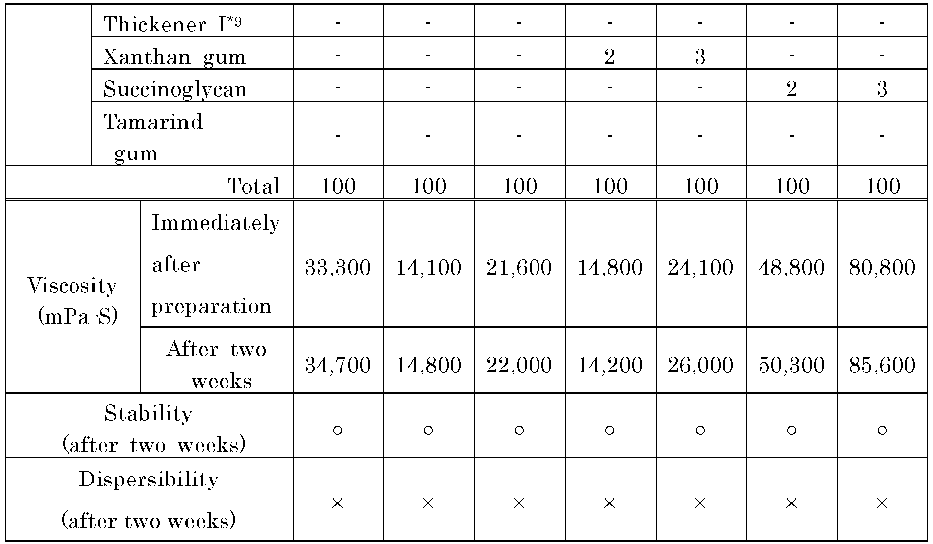

### {Test Example 3}

Compositions were prepared by using a thickener that achieved excellent results in Test Examples 1 and 2, while changing the percentage of the powder and the thickener being added. The viscosity of each composition was measured, and the powder dispersion state was observed. The formations of the prepared compositions are shown in Tables 9 to 11. In Tables 9 to 11, ion-exchanged water was used for the aqueous phase. Synthetic mica was used for the powder. The aforementioned Thickener A was used for the thickener.

The viscosity, pH and results of dispersion stability of the respective compositions are also shown in Tables 9 to 11. The viscosity was measured immediately after preparation of each composition and two weeks after preparation at 30°C with a rotary viscometer (BH-type viscometer from Brookfield) using Rotor No. 6 at a rotation speed of 10 rpm. The evaluation criteria regarding stability and dispersibility were the same as those in the aforementioned Test Examples.

When the content by percentage of the thickener was 0.4% by mass, powder stability could not be achieved. When the content by percentage of the thickener was 0.6 mass%, it was possible to achieve powder stability when the content by percentage of the powder was 40% by mass, but powder stability could not be achieved when the content by percentage of the powder was from 10 to 30% by mass. However, when the content by percentage of the thickener was 0.8% by mass or 1% by mass, it was possible to achieve stability even when the content by percentage of the powder was from 10 to 40% by mass. It is thus considered that, when the content of the thickener is 0.7% by mass or greater, powder stability and dispersibility can be improved, regardless of the content by percentage of the powder.

Noted that, in the composition of the present disclosure, it can be considered that powder stability can, instead, be improved by increasing the content by percentage of the powder. It is considered that, when the content by percentage of the powder is 35% by mass or greater, powder stability and dispersibility can be improved, even in cases where the content of the thickener is from 0.5 to 0.7% by mass.

Since the content by percentage of the powder can be increased, the powder can remain on the skin even if the composition is wiped off after application to the skin.

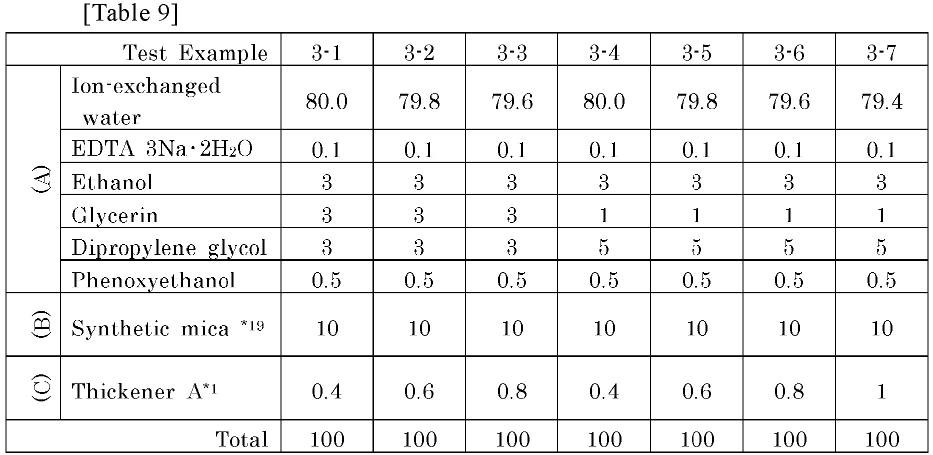

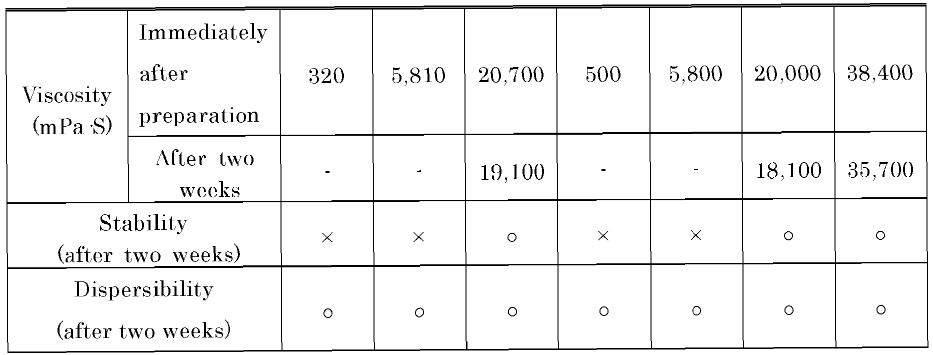

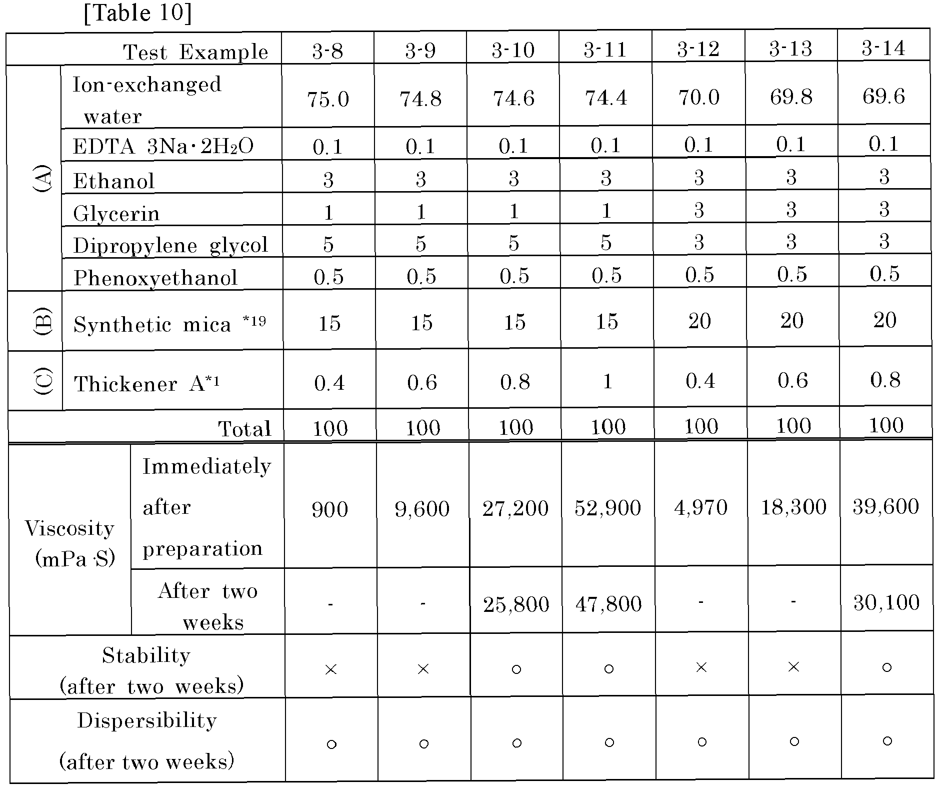

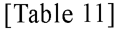

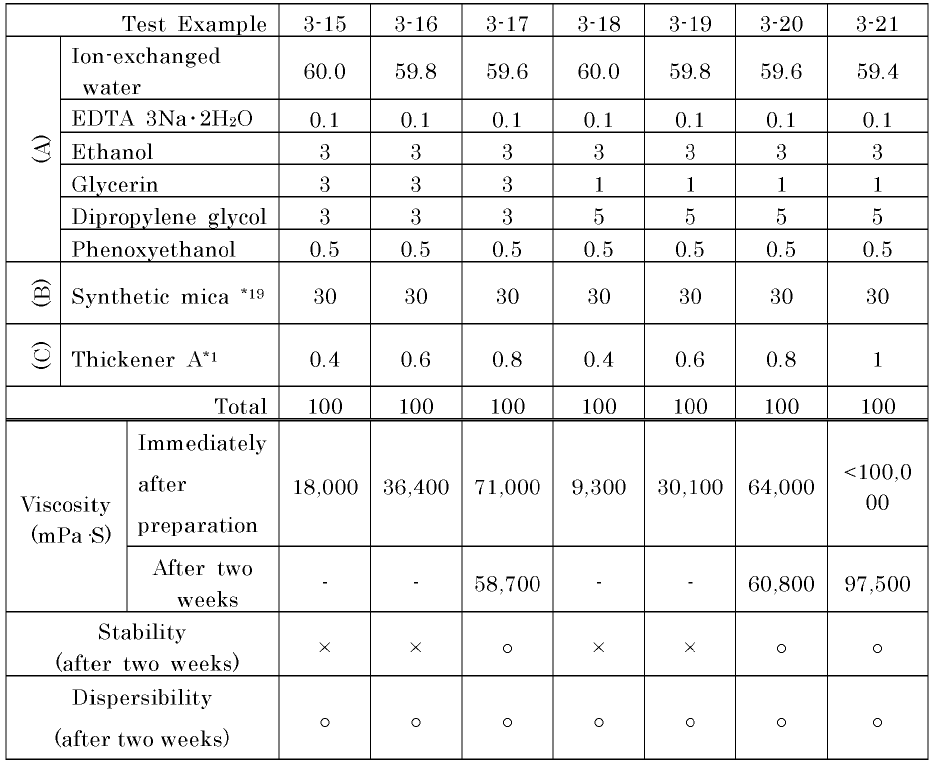

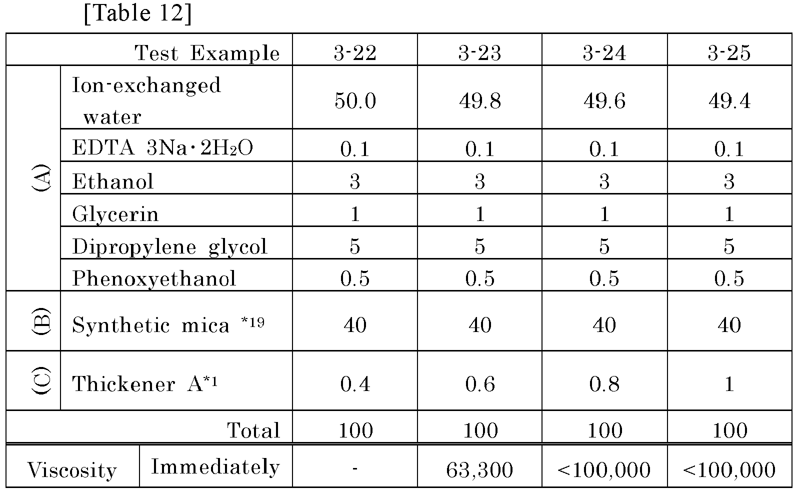

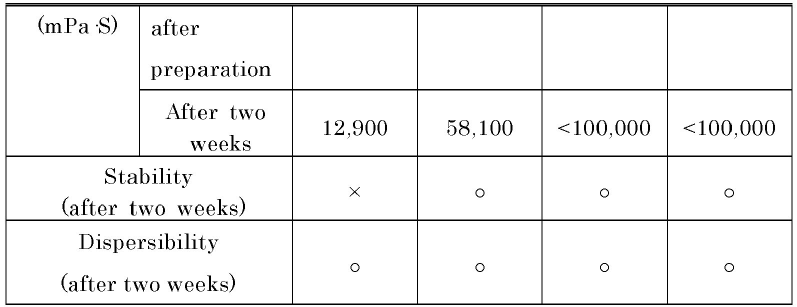

### {Test Example 4}

In order to verify whether powder dispersion stability is affected by the type of powder, compositions were prepared using different types of powders, and the viscosity and stability were verified. The formation of the prepared sample is shown in Table 13. The types of powders used are shown in Tables 14 and 15. Tables 14 and 15 also show the viscosity of each composition immediately after preparation of the sample, the viscosity two weeks after preparation of the sample, and powder stability and dispersibility two weeks after preparation of the sample. The evaluation criteria regarding stability and dispersibility were the same as those in the aforementioned Test Examples. The method for measuring viscosity was the same as that in the aforementioned Test Examples.

Various types of powders were used, such as porous powders, non-porous powders, spherical particles, natural products, synthetic products, surface-treated powders, and metal soaps, but none of the powders caused any problem regarding powder stability and dispersibility. Also, all of the powders were able to achieve high-viscosity compositions. It is thus considered that powder stability and dispersibility are independent of the type of powder.

**[Table 13]**

| | | Example 4 |
|---|---|---|
| (A) | Ion-exchanged water | 49.7 |
| | Ethanol | 15 |
| | Glycerin | 1 |
| | Dipropylene glycol | 3 |
| | EDTA 3Na · 2H₂O | 0.03 |
| | Phenoxyethanol | 0.3 |
| | Citric acid | 0.01 |
| | Sodium citrate | 0.09 |
| (B) | Powder | 30 |
| (C) | Thickener A^{*1} | 0.8 |
| Others | Menthol | 0.03 |
| | Perfume | 0.04 |
| Total | | 100 |

**[Table 14]**

| Test Example | | 4-1 | 4-2 | 4-3 | 4-4 | 4-5 | 4-6 |
|---|---|---|---|---|---|---|---|
| Powder | | Silicone resin *36 | Silica ^{*26} | Crosslink ed polymeth yl methacry late ^{*25} | Natural mica ^{*37} | Synthetic mica *18 | Silicone-t reated mica *38 |
| Visco sity (mP a ·S) | Immediat ely after preparatio n | 56,200 | >100,000 | >100,000 | >100,000 | 57,500 | 41,400 |
| | After two weeks | 54,000 | >100,000 | >100,000 | 84,100 | 54,800 | 43,300 |
| Stability (after two weeks ) | | ○ | ○ | ○ | ○ | ○ | ○ |
| Dispersibility (after two weeks) | | ○ | ○ | ○ | ○ | ○ | ○ |
| *36 KSP100 from Shin-Etsu Silicone | | | | | | | |
| *37: SA310 from Yamaguchi Mica Co., Ltd. | | | | | | | |
| *38: Mica PDM-250 (FS) from Topy Industries Ltd. | | | | | | | |

| | | | | | | | |
|---|---|---|---|---|---|---|---|
| | | | reated talc ^{*28} | oxide ^{*21} | bized titanium oxide ^{*39} | te titanium oxide ^{*22} | nitride ^{*40} |
| Visco sity (mP a ·S) | Immediat ely after preparatio n | >100,000 | 57,000 | 67,500 | 82,900 | >100,000 | 92,800 |
| | After two weeks | >100,000 | 48,100 | 58,800 | 97,300 | >100,000 | 86,500 |
| Stability (after two weeks ) | | ○ | ○ | ○ | ○ | ○ | ○ |
| Dispersibility (after two weeks) | | ○ | ○ | ○ | ○ | ○ | ○ |
| *39: OTS-CR50P from Daitoh Chemical Co., Ltd. | | | | | | | |
| *40: Ronaflair Boroneige SF-12 from Merck | | | | | | | |

### {Test Example 5} (comparative example)

An oil-in-water composition based on the composition disclosed in Patent Literature 2 was prepared, and powder stability and dispersibility were compared. The content by percentage of the powder was varied in Test Examples 5-1 and 5-3 to 5-5. Test Example 5-2 was a composition made by excluding the oil phase from the composition of Test Example 5-1. Table 16 shows the formations and results. The evaluation criteria regarding stability and dispersibility were the same as those in the aforementioned Test Examples (except for stability in Test Examples 5-2 to 5-4).

Test Example 5-1 was able to ensure powder stability and dispersibility even when the content by percentage of the powder was 26% by mass. However, Test Example 5-2, which was made by removing the oil phase from Test Example 5-1, was not able to ensure powder stability and dispersibility. It is thus considered that, in Test Example 5-1, the oil phase contributes to powder stability and dispersibility. It is thus considered that, in the composition of Test Example 5-1, was not able to ensure powder stability and dispersibility. It is thus considered that, in Test Example 5-1, the oil phase contributes to powder stability and dispersibility. It is thus considered that, in the composition of Test Example 5-1, powder stability and dispersibility deteriorate when the content by percentage of the oil phase is 15% by mass or less. Stated differently, it is considered that the composition disclosed in Patent Literature 2 cannot ensure powder stability and dispersibility unless the content by percentage of the oil phase is high. On the other hand, the compositions according to the present disclosure were able to ensure powder stability and dispersibility, even when the content by percentage of the oil phase was 15% by mass or less, and particularly even when no oil phase was present, as shown by Test Examples 1 to 4.

In Test Examples 5-3 to 5-5 in which the content by percentage of the powder exceeded 30% by mass, the oily components floated up and thus powder stability could not be achieved, and also, powder dispersibility could not be achieved. It is thus considered that the composition disclosed in Patent Literature 2 cannot maintain stability and dispersibility unless the content by percentage of the powder is less than 30% by mass. On the other hand, the compositions according to the present disclosure were able to ensure powder stability and dispersibility, even when the content by percentage of the powder was from 30 to 40% by mass, as shown by Test Examples 3 and 4.

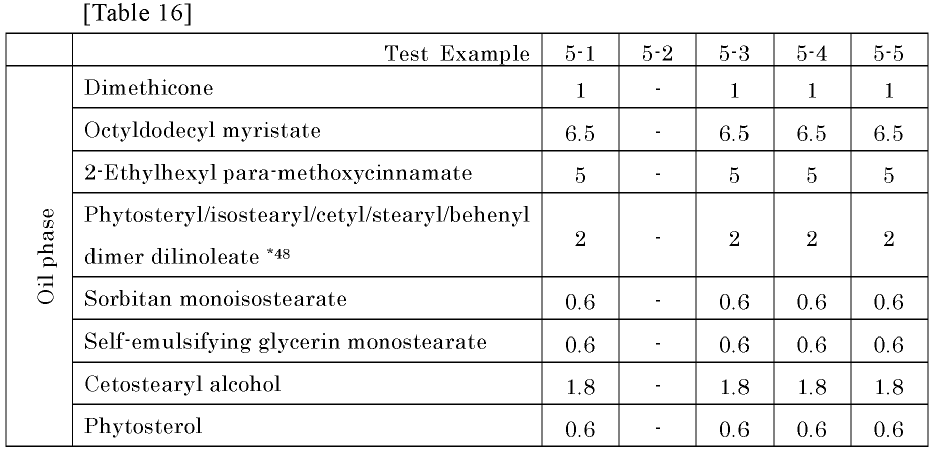

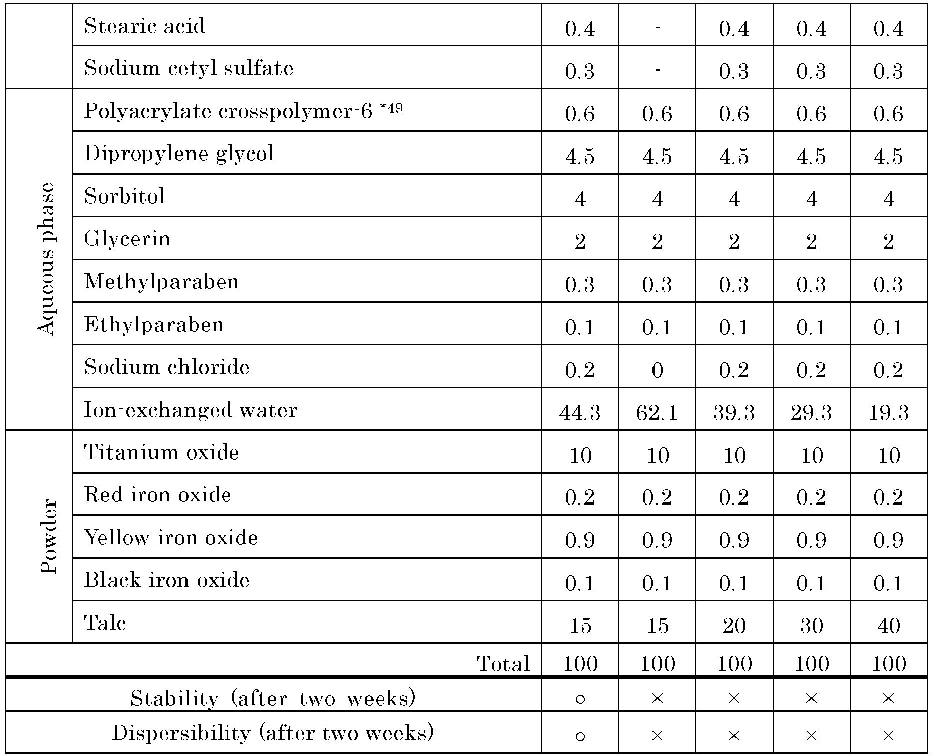

Formulation examples of the powder-containing water-based composition of the present disclosure are described below. However, the application examples of the powder-containing water-based composition of the present disclosure are not limited by the following formulation examples. The unit employed for indicating the contents by percentage of the components shown in the Tables is percent by mass (mass%).

### {Formulation Example 1: Water-based eye shadow (Table 17)}

A water-based eye shadow was prepared using the powder-containing water-based composition of the present disclosure. The formation is shown in Table 17. The water-based eye shadow had excellent powder stability and dispersibility. Also, the water-based eye shadow was able to achieve a fresh feel upon use.

**[Table 17]**

| Formulation Example | | 1 |
|---|---|---|
| (A)Aqueous phase | Ion-exchanged water | Balance |
| | Ethanol | 7 |
| | Glycerin | 1 |
| | Dipropylene glycol | 5 |
| | EDTA 2Na ·2H₂O | 0.03 |
| | Phenoxyethanol | 0.3 |
| | Citric acid | 0.02 |
| | Sodium citrate | 0.08 |
| (B)Powder | Pearl pigment | 20 |
| | Red iron oxide ^{*32} | 0.9 |
| | Yellow iron oxide ^{*23} | 0.1 |
| | Black iron oxide | 0.1 |
| (C)Thickener | Thickener A | 0.9 |
| Total | | 100 |

### {Formulation Example 2: Water-based face powder (Table 18)}

A water-based face powder was prepared using the powder-containing water-based composition of the present disclosure. The formation is shown in Table 18. The water-based face powder had excellent powder stability and dispersibility. Also, the water-based face powder was able to achieve a fresh feel upon use, while preventing stickiness from being felt.

**[Table 18]**

| Formulation Example | | 2 |
|---|---|---|
| (A)Aqueous phase | Ion-exchanged water | Balance |
| | Ethanol | 5 |
| | Glycerin | 5 |
| | EDTA 3Na ·2H₂O | 0.03 |
| | Phenoxyethanol | 0.3 |
| | Citric acid | 0.02 |
| | Sodium citrate | 0.08 |
| (B)Powder | Talc | 7 |
| | Synthetic mica | 10 |
| | Crosslinked polymethyl methacrylate | 5 |
| | Boron nitride | 5 |
| (C)Thickener | Thickener A | 1 |
| Total | | 100 |

### {Formulation Example 3: Astringent lotion (Table 19)}

An astringent lotion was prepared using the powder-containing water-based composition of the present disclosure. The formation is shown in Table 19. The astringent lotion had excellent powder stability and dispersibility. Also, the astringent lotion was able to achieve a fresh feel upon use, while preventing stickiness from being felt after application.

**[Table 19]**

| Formulation Example | | 3 |
|---|---|---|
| (A)Aqueous p hase | Ion-exchanged water | Balance |
| | Ethanol | 2 |
| | Glycerin | 5 |
| | Dipropylene glycol | 5 |
| | EDTA 3Na ·2H₂O | 0.03 |
| | Phenoxyethanol | 0.3 |
| | Citric acid | 0.03 |
| | Sodium citrate | 0.07 |
| (B)Powder | Spherical silica | 10 |
| | Powdered silicone resin | 5 |
| (C)Thickener | Thickener B | 0.9 |
| (D)Others | Perfume | 0.2 |
| Total | | 100 |

### {Formulation Example 4: Water-based eau de toilette (Table 20)}

A water-based eau de toilette was prepared using the powder-containing water-based composition of the present disclosure. The formation is shown in Table 20. The perfume was added to the solvent by first impregnating spherical porous silica with the perfume and letting the spherical porous silica absorb the perfume in advance. The water-based eau de toilette had excellent powder stability and dispersibility. Also, the water-based eau de toilette was able to achieve a fresh feel upon use. Since no ethanol was blended, the eau de toilette had a long-lasting scent.

**[Table 20]**

| Formulation Example | | 4 |
|---|---|---|
| (A)Aqueous phase | Ion-exchanged water | Balance |
| | Glycerin | 3 |
| | Dipropylene glycol | 3 |
| | EDTA 3Na ·2H₂O | 0.03 |
| | Phenoxyethanol | 0.3 |
| | Citric acid | 0.02 |
| | Sodium citrate | 0.08 |
| (B)Powder | Spherical porous silica | 18 |
| | Pearl pigment | 2 |
| (C)Thickener | Thickener A | 0.9 |
| (D)Others | Perfume | 5 |
| | Antioxidant | 0.3 |
| Total | | 100 |

### {Formulation Example 5: Water-based sunscreen (water-based sun-block cosmetic) (Table 21)}

A water-based sunscreen was prepared using the powder-containing water-based composition of the present disclosure. The formation is shown in Table 21. The water-based sunscreen had excellent powder stability and dispersibility. Also, the water-based sunscreen was able to achieve a fresh feel upon use, while preventing stickiness from being felt.

**[Table 21]**

| Formulation Example | | 5 |
|---|---|---|
| (A)Aqueous phase | Ion-exchanged water | Balance |
| | Ethanol | 5 |
| | Glycerin | 7 |
| | Dipropylene glycol | 3 |
| | EDTA 3Na ·2H₂O | 0.03 |
| | Phenoxyethanol | 0.3 |
| | Citric acid | 0.01 |
| | Sodium citrate | 0.09 |
| (B)Powder | Hydrophobized titanium oxide | 5 |
| | Particulate titanium oxide | 5 |
| | Powdered nylon | 10 |
| | Powdered silicone resin | 5 |
| (C)Thickener | Thickener A | 1 |
| (D)Others | Octylmethoxycinnamate | 5 |
| | Silicone oil | 1 |
| | Antioxidant | 0.05 |
| Total | | 100 |

### {Formulation Example 6: Water-based paint (Table 22)}

A water-based paint was prepared using the powder-containing water-based composition of the present disclosure. The formation is shown in Table 22. The water-based paint had excellent powder stability and dispersibility. Also, the water-based paint was able to be used as a paint applicable to paper, for example.

**[Table 22]**

| Formulation Example | | 6 |
|---|---|---|
| (A)Aqueous phase | Ion-exchanged water | Balance |
| (B)Powder | Red iron oxide | 40 |
| | Pearl pigment | 5 |
| (C)Thickener | Thickener A | 1 |
| Total | | 100 |

The powder-containing water-based composition and external skin preparation and manufacturing methods thereof of the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

### Industrial Applicability

### Industrial Applicability

The powder-containing water-based composition of the present disclosure is also applicable to such powder-containing products as coating materials, inks, and food products.

## Claims

1. A powder-containing water-based non-emulsified composition, comprising:
(A) an aqueous phase constituting a continuous phase;
(B) 20 to 45% by mass of a powder; and
(C) 0.7% by mass or greater of a thickener, wherein
said thickener is a polymer that includes 2-acrylamido-2-methylpropanesulfonic acid or a salt thereof as a constituent component,
the powder is dispersed in the composition,
the powder is at least one selected from the group consisting of synthetic mica, mica, sericite, talc, silica, barium sulfate, zinc myristate, glass powder, titanium dioxide, zinc oxide, boron nitride, silicone resin powder, polymethyl methacrylate powder, and nylon powder, and
a content of an oily component is 15% by mass or less relative to the mass of the composition.

2. The composition, according to claim 1, wherein:
said thickener includes at least one selected from a group of consisting of ammonium acryloyldimethyltaurate/beheneth-25 methacrylate crosspolymer, ammonium acryloyldimethyltaurate/vinylpyrrolidone copolymer, and ammonium acryloyldimethyltaurate/dimethylacrylamide crosspolymer.

3. The composition, according to claim 1 or 2, wherein:
the content by percentage of said thickener is 3% by mass or less relative to the mass of the composition.

4. The composition, according to any one of claims 1 to 3, wherein:, two weeks after preparation, the composition has a viscosity of 20,000 mPa·s or greater.

5. The composition, according to any one of claims 1 to 4, further comprising:
50% by mass or less of a water-soluble alcohol relative to the mass of the composition.

6. The composition, according to any one of claims 1 to 5, wherein:
the content by percentage of said oily component is 5% by mass or less relative to the mass of the composition.

7. The composition, according to any one of claims 1 to 6, wherein:, in case where said oily component is included, the content by percentage of a surfactant for emulsifying said oily component is 1% by mass or less relative to the mass of the composition.

8. The composition, according to any one of claims 1 to 7, wherein:
the content by percentage of said aqueous phase is 40% by mass or greater relative to the mass of the composition.

9. The composition, according to any one of claims 1 to 8, wherein:
the composition further comprises a volatile oily component;
said powder is porous; and
said powder is impregnated with at least a portion of said oily component.

10. The composition according to any one of claims 1 to 9 in the form of an external skin preparation.

## Patentansprüche

1. Pulverhaltige, nicht emulgierte Zusammensetzung auf Wasserbasis, umfassend:
(A) eine wässrige Phase, die eine kontinuierliche Phase bildet;
(B) 20 bis 45 Massen-% eines Pulvers; und
(C) 0,7 Massen-% oder mehr eines Verdickungsmittels, wobei
das Verdickungsmittel ein Polymer ist, das 2-Acrylamido-2-methylpropansulfonsäure oder ein Salz davon als einen Bestandteil enthält,
das Pulver in der Zusammensetzung dispergiert ist,
das Pulver mindestens eines, ausgewählt aus der Gruppe bestehend aus synthetischem Glimmer, Glimmer, Sericit, Talkum, Siliciumdioxid, Bariumsulfat, Zinkmyristat, Glaspulver, Titandioxid, Zinkoxid, Bornitrid, Silikonharzpulver, Polymethylmethacrylatpulver und Nylonpulver, ist und
der Gehalt eines öligen Bestandteils 15 Massen-%oder weniger, bezogen auf die Masse der Zusammensetzung, beträgt.

2. Zusammensetzung nach Anspruch 1, wobei:
das Verdickungsmittel mindestens eines, ausgewählt aus einer Gruppe bestehend aus Ammoniumacryloyldimethyltaurat/Beheneth-25-Methacrylat-Kreuzpolymer, Ammoniumacryloyldimethyltaurat/Vinylpyrrolidon-Copolymer und Ammoniumacryloyldimethyltaurat/Dimethylacrylamid-Kreuzpolymer, enthält.

3. Zusammensetzung nach Anspruch 1 oder 2, wobei:
der prozentuale Gehalt des Verdickungsmittels 3 Massen-% oder weniger, bezogen auf die Masse der Zusammensetzung, beträgt.

4. Zusammensetzung nach einem der Ansprüche 1 bis 3, wobei: die Zusammensetzung zwei Wochen nach der Herstellung eine Viskosität von 20.000 mPa s oder mehr aufweist.

5. Zusammensetzung nach einem der Ansprüche 1 bis 4, ferner umfassend:
50 Massen-% oder weniger eines wasserlöslichen Alkohols, bezogen auf die Masse der Zusammensetzung.

6. Zusammensetzung nach einem der Ansprüche 1 bis 5, wobei:
der prozentuale Gehalt des öligen Bestandteils 5 Massen-% oder weniger, bezogen auf die Masse der Zusammensetzung, beträgt.

7. Zusammensetzung nach einem der Ansprüche 1 bis 6, wobei: für den Fall, dass der ölige Bestandteil enthalten ist, der prozentuale Gehalt eines Tensids zum Emulgieren des öligen Bestandteils 1 Massen-% oder weniger, bezogen auf die Masse der Zusammensetzung, beträgt.

8. Zusammensetzung nach einem der Ansprüche 1 bis 7, wobei:
der prozentuale Gehalt der wässrigen Phase 40 Massen-% oder mehr, bezogen auf die Masse der Zusammensetzung, beträgt.

9. Zusammensetzung nach einem der Ansprüche 1 bis 8, wobei:
die Zusammensetzung ferner einen flüchtigen öligen Bestandteil enthält;
das Pulver porös ist; und
das Pulver mit mindestens einem Teil des öligen Bestandteils imprägniert ist.

10. Zusammensetzung nach einem der Ansprüche 1 bis 9 in Form eines äußerlichen Hautpräparats.

## Revendications

1. Composition non émulsifiée, à base d'eau, contenant de la poudre, comportant :
(A) une phase aqueuse constituant une phase continue ;
(B) 20 à 45 % en masse d'une poudre ; et
(C) 0,7 % en masse ou plus d'un épaississant, dans laquelle
ledit épaississant est un polymère qui inclut de l'acide 2-acrylamido-2-méthylpropanesulfonique ou un sel de celui-ci en tant que composant constitutif,
la poudre est dispersée dans la composition,
la poudre est au moins un élément choisi parmi le groupe constitué de mica de synthèse, de mica, de séricite, de talc, de silice, de sulfate de baryum, de myristate de zinc, de poudre de verre, de dioxyde de titane, d'oxyde de zinc, de nitrure de bore, de poudre de résine de silicone, de poudre de poly(méthacrylate de méthyle) et de poudre de nylon, et
une teneur en un composant huileux est de 15 % en masse ou moins par rapport à la masse de la composition.

2. Composition selon la revendication 1, dans laquelle :
ledit épaississant inclut au moins un élément choisi parmi un groupe constitué d'un polymère réticulé d'acryloyldiméthyltaurate d'ammonium/béhéneth-25 méthacrylate, d'un copolymère d'acryloyldiméthyltaurate d'ammonium/vinylpyrrolidone et d'un polymère réticulé d'acryloyldiméthyltaurate d'ammonium/diméthylacrylamide.

3. Composition selon la revendication 1 ou 2, dans laquelle :
la teneur en pourcentage dudit épaississant est de 3 % en masse ou moins par rapport à la masse de la composition.

4. Composition selon l'une quelconque des revendications 1 à 3, dans laquelle, deux semaines après préparation, la composition a une viscosité de 20 000 mPa·s ou plus.

5. Composition selon l'une quelconque des revendications 1 à 4, comportant en outre 50 % en masse ou moins d'un alcool soluble dans l'eau par rapport à la masse de la composition.

6. Composition selon l'une quelconque des revendications 1 à 5, dans laquelle :
la teneur en pourcentage dudit composant huileux est de 5 % en masse ou moins par rapport à la masse de la composition.

7. Composition selon l'une quelconque des revendications 1 à 6, dans laquelle, dans un cas où ledit composant huileux est inclus, la teneur en pourcentage d'un tensioactif destiné à émulsifier ledit composant huileux est de 1 % en masse ou moins par rapport à la masse de la composition.

8. Composition selon l'une quelconque des revendications 1 à 7, dans laquelle :
la teneur en pourcentage de ladite phase aqueuse est de 40 % en masse ou plus par rapport à la masse de la composition.

9. Composition selon l'une quelconque des revendications 1 à 8, dans laquelle :
la composition comporte en outre un composant huileux volatil ;
ladite poudre est poreuse ; et
ladite poudre est imprégnée d'au moins une partie dudit composant huileux.

10. Composition selon l'une quelconque des revendications 1 à 9 se présentant sous la forme d'une préparation externe pour la peau.
